# EUROPEAN PATENT SPECIFICATION

(11) **EP 2 072 072 B1**
(45) Date of publication and mention of the grant of the patent: **24.08.2016**
(21) Application number: 07024466.0
(22) Date of filing: 18.12.2007
(51) Int. Cl.: A61M 5/145

(54) **Method and device for micro-dosing a liquid**
Verfahren und Vorrichtung zur Mikrodosierung einer Flüssigkeit
Procédé et dispositif pour le micro-dosage d'un liquide

(43) Date of publication of application: 24.06.2009
(73) Proprietor: F.Hoffmann-La Roche AG, 4070 Basel (CH); Roche Diabetes Care GmbH, 68305 Mannheim (DE)
(72) Inventor: Haenggi, Roger, 4208 Nunningen (CH); Niklaus, Hanspeter, 4853 Riken (CH)
(74) Representative: Rentsch Partner AG

(56) References cited:
- WO-A-01/83005
- WO-A-02/30554
- WO-A-03/099357
- US-A- 4 634 431
- US-A1- 2001 034 506

## Description

The present invention relates to a method of and a portable device for micro-dosing a liquid and to the use of this method and this device for dispensing a liquid drug according to the preambles of the independent claims.

Micro-dosing of liquid substances, i.e. dosing of liquid substances in the range of micro-liters, is typically applied in the pharmaceutical and chemical industry and in particular in medical applications like e.g. the administration of liquid drugs to a patent's body.

In particular in the treatment of diabetes and in certain pain treatments, in which the patient has a continuous and during the day varying demand of a medicament that can be administered subcutaneously, portable computer controlled micro dosing devices are employed, e.g. insulin pumps, which typically contain a drug amount suitable for several days of treatment in a drug reservoir and supply the drug to the patient's body according to a predetermined daily profile via an infusion cannula.

However, specifically in the before mentioned applications where the therapy takes place in normal life, there is an increasing desire to invisibly carry the dosing device. Thus, it is of utmost importance that it is as small as possible in order not to be noticeable through the clotting when worn at the patient's body and at the same time provide a sound wearing comfort.

The outer dimensions of today's portable micro-dosing devices for liquid drugs are mainly dictated by the space requirements of the liquid reservoir and of the drive unit of the device. Typically, as drug reservoir, a disposable vial with a displacement piston is employed and a discharge of drug is accomplished by moving said piston towards the discharge end of the vial, thereby displacing liquid drug out of the vial.

In US 6,248,093 B1, several embodiments of portable micro dosing devices with different drive units and different arrangements of the reservoir and the drive unit are disclosed. However, in the embodiments where the drive motor of the drive unit is arranged beside the reservoir, an additional transmission gear is required, which causes additional costs, friction and space requirements and is furthermore prone to wear and tear.

In a further embodiment shown in US 6,248,093 B1, the drive motor is arranged in line with the liquid reservoir and a threaded appendix of the reservoir piston, which engages with its thread a gear wheel of the drive motor and is thereby linearly displaceable by the drive motor for pushing the piston of the reservoir, in a retracted position axially extends substantially over the entire length of the drive motor at the outer periphery thereof, resulting in a relative compact design..However, the threaded appendix at its side facing away from the gear wheel of the drive motor needs to be supported, which causes, in the present case where a backlash free arrangement is necessary in order to ensure precise dosing, considerable friction. Furthermore, the transmission of forces into the piston is extremely asymmetrical, resulting in a design which is prone to tilting of the piston and which furthermore promotes impreciseness caused by deformation of parts under load.

In yet a further embodiment shown in US 6,248,093 B1, the drive unit consists of a drive motor with a reduction gear, both arranged in line with the reservoir. A threaded sleeve, which engages a gear wheel of the drive unit and is thereby linearly displaceable by the drive unit for actuating the piston of the reservoir, in a retracted position completely circumferentially surrounds the reduction gear but not the drive motor, resulting in a design where the length of the motor adds to the length of the device and the threaded bushing dictates with its outer diameter the minimum inner diameter of the reservoir. Thus, even though from a mechanical point of view this design avoids the disadvantages of the before mentioned embodiments, it is unsatisfactory with respect to the length and the thickness of the device.

In WO 02/30554 A2 a micro dosing device is disclosed with a drive unit consisting of a drive motor, the driving shaft of which is at one end of the motor connected to a sleeve which, at its outer circumference, provides an outer thread which meshes with a corresponding inner thread in the housing of the device. Upon an activation of the drive motor, the sleeve is rotated and thereby is linearly displaced in the housing, while the motor, which in a linearly displaceable but noun-rotatable is received in the housing, axially travels together with the sleeve. Inside the sleeve, a syringe type liquid reservoir is received in such a manner that, upon the rotation and linear displacement of the sleeve in the housing, the liquid reservoir is rotated together with the sleeve and its piston is pushed towards its discharging opening, thereby displacing liquid from the liquid reservoir. Also this design is unsatisfactory with respect to the length and the thickness of the device and furthermore provides the disadvantage that the liquid reservoir is rotated so that a complex and complicated interconnection between the reservoir and a discharge connector is required when the device shall be coupled to a rotationally stationary catheter. Such an interconnection is furthermore complicated since it is prone to leakage and can furthermore result in a contamination of the liquid discharged from the reservoir with particles generated through friction between stationary and rotating components.

Hence, it is the object of the invention to provide a method of and a portable device for micro-dosing a liquid, which at least partially avoid the disadvantages of the above mentioned prior art.

This object is achieved by the method and device according to the independent claims.

A first aspect of the invention concerns a method of micro-dosing a liquid from a liquid reservoir which is having a displacement piston arranged in its inside, e.g. a reservoir of the vial-type, by axially moving this piston within the reservoir towards the discharge end thereof, thereby displacing liquid out of said reservoir. The method is preferably conducted in a manner that a time controlled dispensing of liquid results, i.e. the liquid is dispensed according to a specific profile over time, e.g. according to a pre-selected daily profile. As already mentioned in the introductory part, micro-dosing of liquid substances means a controlled dispensing of liquid substances in the range of micro-liters, as is typically applied in the pharmaceutical and chemical industry and in particular in medical applications like e.g. the administration of liquid drugs like insulin to a patient's body.

In one step of the method, a portable structure for receiving the liquid reservoir is provided.

Furthermore, a drive unit for generating the forces for axially moving the displacement piston of the liquid reservoir is provided. The drive unit features a first end and a second end and comprises a drive motor. By activating the drive motor, it is axially displaceable relative to the structure.

In yet a further step of the method, the second end of the drive unit is coupled to the piston of the liquid reservoir and the liquid reservoir is secured to said structure so that in displacement direction of its piston it is immobile relative to the structure.

In yet a further step of the method, the drive motor is activated so that the drive unit is axially moved towards the piston of said reservoir, with the effect that the piston is axially moved within the reservoir towards the discharge end thereof, thereby causing liquid to be dispensed out of the reservoir.

The axial displacement of the drive unit is effected by rotating with the drive unit a first member having an outer thread, which first member is coupled to said drive unit. This outer thread engages a thread of a second member, which is integrally formed by or secured to the structure of the device in such a manner that in axial direction of the rotational axis of the first member facing away from the reservoir and rotational around said axis it is immobile relative to the structure.

Between the drive unit and the second member, an anti-rotation element is arranged which prevents a rotation of the drive unit relative to the second member but permits axial movement of the drive unit relative to these elements.

By micro-dosing of liquid from a vial-type reservoir according to this method, the individual elements required to conduct the method can be arranged in an ultra compact fashion without compromising on preciseness and reliability, thus, this method promotes the design of ultra compact portable micro-dosing devices for liquid medicaments, in particular insulin. Furthermore, a well proven and sturdy mechanism for effecting the axial movement of the drive unit is arrived at, which allows for sensitive displacement control, e.g. by way of a control unit with an encoder coupled to the drive shaft of the drive motor and, in particular in embodiments where the device comprises a second element with a sleeve-like design, allows to encapsulate all mechanical parts for effecting the axial displacement of the drive unit inside the second member, thereby forming a modular element. Last but not least, this method offers the possibility to connect in a cost effective, uncomplicated and reliable manner a rotationally stationary catheter to the liquid reservoir for receiving liquid from it.

In a preferred embodiment of the method the second member employed provides an inner thread for engagement with the thread of the first member. Furthermore, the drive unit for dispensing liquid from the reservoir is axially displaced from a retracted position, in which its second end, which is coupled to the piston of the reservoir, is closest to the inner thread of the second member - this is typically the position when a new, completely filled reservoir is received in the structure and is coupled to the second end of the drive unit - to an extended position, in which the second end of the drive unit is furthermost from the thread of said second member - this is typically the position when the piston has fully been pushed towards the discharge end of the reservoir, i.e. the reservoir has fully been emptied with the device - in such a manner that the first end of the drive unit, at least in the retracted position, is at least partly circumferentially surrounded by the inner thread of the second member. This means that at least in the retracted position at least at certain positions distributed at the circumference of the drive unit at least over an angle of more than 180° (360° is the complete circumference of the drive unit) portions of the inner thread of the second member are facing towards the drive unit. This is the case e.g. where two areas of the inner thread of the second member, which each circumferentially extend over a certain angle (e.g. 5°) of the circumference of the first end of the drive unit, are positioned opposite to each others (180°) with the first end of the drive unit arranged between them, e.g. where three areas of said inner thread are equally distributed over the circumference of the first end of the drive unit or e.g. where the inner thread consists of one area only, which covers an angle of more than 180° of circumference of the first end of the drive unit. By this, the drive unit with its driven first member together with the second member forms a telescopic drive mechanism having excellent force transmission properties and thus being sturdy and reliable.

In a further preferred embodiment of the method, the activity of said drive unit is monitored by means of an encoder arranged at said drive unit with an associated control system, in order to establish a closed loop control of the displacement position of the drive unit relative to the structure and therefore of the amount of liquid dispensed. In cases where the drive motor is a rotating motor, it is preferred to monitor with the encoder the total or relative number of revolutions or a total or relative angle of rotation of the drive shaft of the motor. In cases where a linear motor is employed, the absolute or relative linear displacement of the actuator the motor or directly of the drive unit may be monitored.

In yet a further preferred embodiment of the method, the forces exerted to the piston of the liquid reservoir are monitored with a force sensor with associated control unit in order to detect an operational disorder, like e.g. an occlusion in the liquid path downstream of the reservoir or e.g. a tilted piston, or an empty liquid reservoir.

In yet a further preferred embodiment of the method, the power for operating the drive motor is supplied via one or several anti-rotation elements, which are arranged between the drive unit and the second member for preventing a rotation of the drive unit relative to the second member and to the structure. This can be accomplished e.g. by means of wires, by so called flexprints or by sliding contacts established between components of the anti-rotation elements formed by the drive unit and components thereof formed by the second member or the structure, respectively. By means of this, transmittal of the energy for the drive can be achieved in a simple and reliable manner.

In yet a further preferred embodiment of the method, the drive motor is, according to a specific activity profile, activated by an electronic control system, for the time-controlled dispensing of liquid from the liquid reservoir. In the preferred application of the method for dispensing a liquid medicament, like e.g. insulin, for the treatment of a disease, like e.g. diabetes, this is done according to a daily dispensing profile which may however differ from day to day, e.g. be different over the weekend compared to the normal working days. Where, as before described, encoders and/or force sensors are employed, these are functionally connected to the control system in order to establish a closed loop control and/or respective alarm functions.

A second aspect of the invention concerns a portable device, preferably for performing the method according to the first aspect of the invention, for micro-dosing of a liquid from a liquid reservoir, which in a releasable manner is received inside the device, by axially moving a displacement piston arranged in the reservoir and thereby displacing liquid out of it. Preferably, the reservoir is a vial containing a liquid medicament, like e.g. insulin.

The device comprises a structure for receiving the liquid reservoir and a drive unit for generating the forces for axially moving the piston, with a first end and a second end and with a drive motor. The drive unit is axially displaceable relative to the structure by activating its drive motor. At its second end, the drive unit comprises coupling means for coupling it, in a releasable manner in order to be suitable for multiple use, to the piston of the liquid reservoir in order to axially move said piston in the reservoir towards an discharge opening of the reservoir upon an axial movement of the drive unit and thereby dispensing liquid.

The device further comprises a rotatable first member, which defines an axis of rotation and has an external thread coaxially arranged around said axis of rotation, and a second member, which is integrally formed by or is secured to the structure of the device. This second member has a thread which engages the outer thread of the first member. The first member is coupled to the drive unit at the first end thereof in such a manner that, upon activation of the drive motor of the drive unit, it is rotated around its axis of rotation, thereby axially moving itself and the drive unit relative to the second member and to the structure of the device.

Between the drive unit and the second member there is arranged-an anti-rotation element which prevents a rotation of the drive unit relative to the second member but permits axial movement of the drive unit relative to these elements.

This concept according to the invention allows the provision of ultra compact portable devices for micro-dosing liquid medicaments from vial-type reservoir, with a well proven and sturdy mechanism for effecting the axial displacement of the drive unit is provided which furthermore is well suited for precise displacement control, e.g. by way of an encoder coupled to the drive shaft of the drive motor with an associated control. It furthermore offers the advantage that, in particular in embodiments where the device comprises a second element with a sleeve-like design, all mechanical parts for effecting the axial displacement of the drive unit can be encapsulated inside the second member, thereby forming a modular element. Last but not least, this device offers the possibility to connect in a cost effective, uncomplicated and reliable manner a rotationally stationary catheter to the liquid reservoir of the device for receiving liquid from it.

In a preferred embodiment of the device, the thread of the second member is an inner thread. Furthermore it is preferred that the first member and the drive unit, by activating the drive motor and subsequent rotation of the first member, can axially be displaced relative to the second member and the structure from a retracted position to an extended position. As retracted position is regarded a position in which the second end of the drive unit - the end which is supposed to be coupled to the piston of the reservoir - is closest to the inner thread of the second member, which typically is the case when a new, completely filled reservoir will be installed within the device. As extended position a position is regarded in which the second end of the drive unit is farthest away from the inner thread of the second member, which typically is the case when the reservoir has been emptied by means of the device. In the retracted-position, the second member with its inner thread at least partly circumferentially surrounds at least the first end of the drive unit. As already mentioned earlier, this means that in the retracted position and possibly also in further positions between the retracted and the extended position at least at certain positions distributed at the circumference of the first end of the drive unit over an angle of more than 180° (360° is the complete circumference of the drive unit), portions of the inner thread of the second member are facing towards the first end of the drive unit. This is the case e.g. where two areas of the inner thread of the second member, which each cover a certain angle of circumference of the first end of the drive unit (e.g. 5°), are positioned opposite to each others (180°) with the first end of the drive unit arranged between them, e.g. where three areas of said inner thread are equally distributed over the circumference of the first end the drive unit or e.g. where the inner thread consists of one area only which covers an angle of more than 180° of circumference of the first end of the drive unit. By this, the drive unit with its driven first member together with the second member forms a telescopic drive mechanism having excellent force transmission properties and thus being sturdy and reliable.

In the before mentioned preferred embodiment of the device it is furthermore preferred that the inner thread of the second member in the retracted position at least partly circumferentially surrounds, in the fashion described before, not only the first end of the drive unit but at least half of the length of the drive unit and preferably substantially the entire length of the drive unit. By this, the drive unit with its driven first member together with the second member forms a telescopic drive mechanism having an excellent ratio between retracted and extended length.

In a further preferred embodiment of the device, the second member is a sleeve-like element forming the internal thread at its inside. Out of one end of this sleeve-like element, the second end of the drive unit protrudes, in the retracted as well as in the extended position. By this, the drive unit with its driven first member can be received inside the second member to form together with the second member a compact, enclosed telescopic drive mechanism which can be provided as a module.

In the before mentioned preferred embodiment it is furthermore preferred that between the end of the second member, out of which the second end of the drive unit protrudes, and the drive unit there are arranged sealing means, like e.g. an O-ring, in order to prevent dirt or liquids to enter into the second member.

In yet a further preferred embodiment of the device, the drive unit further comprises a reduction gear arranged between the drive motor and the first member. By this, relative small motors can be employed without compromising on the torque available for rotating the first member and a sensitive displacement control becomes possible. Furthermore, in this case it is preferred that this gear forms the first end of the drive unit, thus, is physically located between the drive motor and the first member. By this, the drive unit can be designed as slim as possible.

In yet a further preferred embodiment of the device, the drive motor has a drive shaft defining an axis of rotation. Preferably, in the embodiments mentioned earlier with the first member, this axis of rotation and the axis of rotation of said first member are coaxially aligned, further promoting a slim and compact design.

In yet a further embodiment of the device, the drive unit is equipped with an encoder with associated control means for monitoring the activity of the drive unit in order to establish a closed loop control of the displacement position of the drive unit relative to the structure and therefore of the amount of liquid dispensed. In cases where the drive motor is a rotating motor, it is preferred to monitor with the encoder the total or relative number of revolutions or a total or relative angle of rotation, respectively, of the shaft of the motor. In cases where a linear motor is employed, the absolute or relative linear displacement may directly be monitored.

In the before mentioned preferred embodiment it is furthermore preferred that the encoder itself is arranged at the second end of the drive unit, preferably at least partially arranged inside a protrusion which is, when the piston of the liquid reservoir is coupled to the second end of the drive unit, at least in part received inside said piston. By this, an encoder can be used without necessarily increasing the length of the device.

In the before mentioned embodiment it is furthermore preferred that the power for operating said drive motor is supplied via said anti-rotation elements to said drive motor. This can be accomplished e.g. by means of wires, by so called flexprints or by sliding contacts established between components of the anti-rotation elements. By means of this, transmittal of the energy for the drive can be achieved in a simple and reliable manner.

In yet a further preferred embodiment, the device comprises a force sensor with associated controls to permit monitoring of the forces that in operation are exerted to the piston of the liquid reservoir. By this, an operational disorder, like e.g. an occlusion in the liquid path downstream of the reservoir or e.g a tilted piston, can be detected or an empty liquid reservoir. In embodiments of the device with the second member, it is preferred to axially support said second member by the force sensor in order to permit determination of the forces. In other embodiments it is e.g. envisaged to include the force sensor in the coupling means of the drive unit.

In yet a further embodiment of the device it is preferred that the axis of rotation of said first member is coaxially aligned with a central axis of the piston of the liquid reservoir which in operation is received in the device. By this, the advantage is arrived at that a fully symmetrical transmission of forces from the first member to the piston becomes possible, thus any tendency for bending and tilting of the involved components is reduced to a minimum and therewith also the friction between these parts and adjacent stationary parts when moved relative to each other.

In yet a further preferred embodiment the device further comprises computerized control means for controlling said drive unit in such a manner that a time-controlled dispensing of liquid is possible, i.e. the drive motor is activated according to a specific activity profile by an electronic control system, for the time-controlled dispensing of liquid from the liquid reservoir. In the preferred application of the method for dispensing a liquid medicament, like e.g. insulin, for the treatment of a disease, like e.g. diabetes, this is done according to a daily dispensing profile which may however differ from day to day, e.g. be different over the weekend compared to the normal working days. Where, as described before, encoders and/or force sensors are employed, these are functionally connected to the control system in order to establish a closed loop control and/or respective alarm functions.

In yet a further preferred embodiment, the device further comprises a liquid reservoir with a displacement piston received in its structure and coupled to the second end of the drive unit. Preferably, this reservoir contains a liquid drug, like e.g. insulin or a liquid pain killer.

Preferably, the method according to the first aspect of the invention or the device according to the second aspect of the invention are used for dispensing of a liquid drug, in particular insulin or a liquid pain killer. In these applications, the advantages of the invention become especially clearly apparent.

Further preferred embodiments of the invention become apparent from the dependent claims and from the following description by way of the drawings. Therein show:
Fig. 1a a sectional view through a portion of an infusion pump according to the invention with completely filled reservoir;
Fig. 1b a cut along line C-C in Fig. 1a; and
Fig. 2 an illustration as in Fig. 1a with partially emptied reservoir.

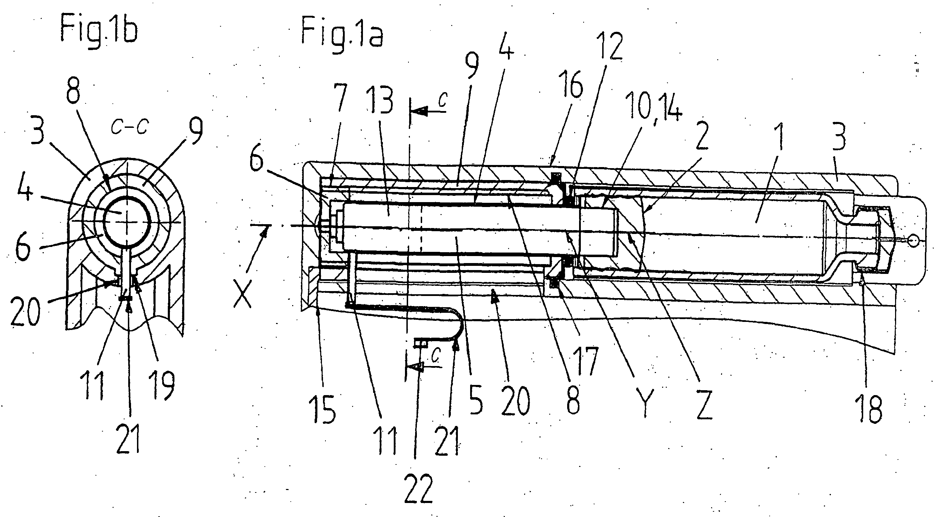

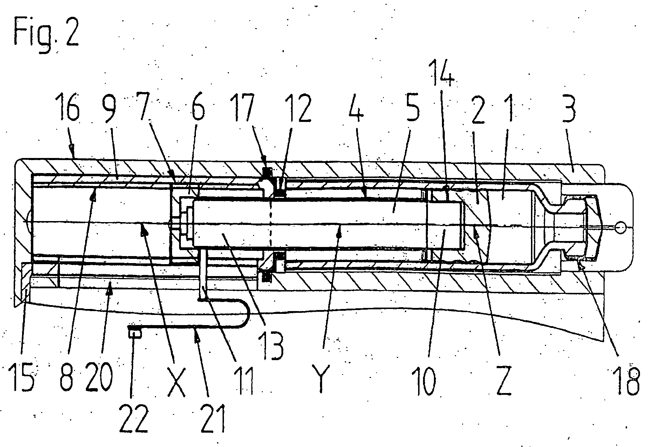

The mechanical portion (pump section) of a micro-dosing device according to the invention in the form of a portable infusion pump for the time controlled administration of insulin is illustrated in the Figures 1a, 1b and 2, once in longitudinal section with the pump being equipped with a new, completely filled vial 1 of insulin (Fig. 1a), once in longitudinal section with the pump being equipped with a used, only partially filled vial 1 of insulin (Fig. 2) and onces in section along line C-C in Fig. 1a (Fig. 1b). As can be seen from these drawings, the infusion pump comprises a structure 3 forming the housing 16 of the pump in which a drive unit 4 and a vial 1 containing insulin are arranged in line. The vial 1 includes a displacement piston 2 with a central axis Z, which can be axially moved inside the vial 1 towards the discharge end 18 thereof for displacing insulin out of the vial 1. The drive unit 4 comprises a rotational electric drive motor 5 with a drive shaft (not shown) defining an axis of rotation Y and a reduction gear 13, which at its power intake side is coupled to the drive shaft of the drive motor 5 and at its power output side is coupled to a gear wheel 6 (the first member according to the claims) defining an axis of rotation X and having an outer thread 7 arranged around this axis X. The gear wheel 6, the gear 13 and the drive motor 5 are arranged in line so that the axis X of the gear wheel 6 and the axis Y of the drive motor 5 are coaxially aligned, wherein the gear 13 is located between the gear wheel 6 and the drive motor 5. By this arrangement; the end of the drive unit 4 facing away from the vial 1 (the first end according to the claims) is formed by the reduction gear 13. At the other end of the drive motor 5, an encoder 10 is arranged inside an end portion 14 of the housing of the drive unit 4 (the second end according to the claims), which protrudes inside the displacement piston 2 of the vial 1 and thereby at the same time forms coupling means 14 according to the claims by which said piston 2 is coupled in a releasable manner to this end of the drive unit 4.

The outer thread 7 of the gear wheel 6 engages an inner thread 8 of a threaded sleeve 9 surrounding same, which sleeve 9 is secured to the structure 3 of the infusion pump in such a manner that in axial direction facing away from the vial 1 and rotationally around the axis X of the gear wheel 6 it is immobile with respect to the structure 3. For accomplishing this, the sleeve 9 in axial direction abuts onto a force sensor 15 arranged at the bottom of a cavity provided in the structure 3 for receiving the sleeve 9 (see Figures 1a and 2), while rotationally it comprises at its outer periphery a radially protruding rim 19 which engages a corresponding groove in the structure 3 (see Fig. 1b). The drive unit 4 at its outer periphery comprises a finger 11, which radially extends through a longitudinal slot 20 formed in the sleeve 9 and in the structure 3, thereby forming together with the slot 20 an anti-rotating element according to the claims which prevents a rotation of the drive unit 4 relative to the sleeve 9 but permits axial travel of the drive unit 4 relative to the sleeve 9.

As can be seen when considering the Figures 1a and 2 in combination, by activating the drive motor 5, the gear wheel 6 is rotated by the reduction gear 13 around its axis X and thus travels with its outer thread 7 in axial direction along the inner thread 8 of the sleeve 9. By doing so, it carries with it the drive unit 4 and the displacement piston 2 coupled to the end thereof and displaces them relative to the sleeve 9 and to the structure 3 and thus to the vial 1, thereby pushing the piston 2 inside the vial 1 towards the discharge end 18 thereof and dispensing insulin from the vial 1. Fig. 2 shows a state in which the piston 2 has already travelled two thirds of its design travel way, i.e. two thirds of the rated volume of insulin of the vial 1 have already been dispensed.

As becomes apparent when looking at Fig. 1b, the end of the drive unit 4 carrying the gear wheel 6 is, with the only exception of the slot 20 receiving the finger 11, completely enclosed by the inner thread 8 of the sleeve 9, i.e. this thread 8 circumferentially surrounds this end of the drive unit 4 by nearly 360°.

As can further be taken from the Figures 1a and 2, the before mentioned end of the drive unit 4 carrying the gear wheel 6 in any displacement position of the drive unit 4 remains within the threaded area 8 of the sleeve 9, and in the fully retracted state (the retracted position according to the claims) shown in Fig. 1a surrounds the drive unit 4 with its inner thread 8 nearly over its complete length, while merely the end portion of the drive unit 4 which is coupled to the piston 2 of the vial 1 protrudes out of said sleeve 9.

In order to prevent dirt and liquid from entering the sleeve 9 and the area between the sleeve 9 and the structure 3, O-rings 12, 16 are provided as sealing elements.

Furthermore, the infusion pump comprises a computerised control unit (not shown) with display, user interface, power supply and control board, to which the encoder 10 and the force sensor 15 report and by which the drive unit 4 is controlled to precisely dispense insulin from the vial 1 according to a predefined daily profile.

The electrical connection between the stationary and the movable, electrical parts of the device, i.e. between the control unit and the drive motor 5 with the encoder 10, in this embodiment is established via a flexprint 21, which compensates the relative movement between these parts during operation. The flexprint 21, which provides several electrical lines, is mechanically connected with one of its ends to the free end of the finger 11, while its electrical lines are electrically connected with corresponding electrical lines which are arranged inside the finger 11 and are leading to the drive motor 5 and to the encoder 10. The other end of the flexprint 21 is connected mechanically and electrically by means of a connector 22 to the control board (not shown).

In the current case, in which a brushless DC-motor is employed, the flexprint 21 contains eight electrical lines. Three of these lines are connected to the encoder 10, which comprises hall sensors, to control the angular position of the motor shaft. Further three lines are connected to the three motor windings (three-phased motor arrangement), one line is a power line, which in this embodiment is set to 3 volts, and the last line is a connection to ground.

In further preferred embodiments, the flexprint 21 is arranged directly at the drive unit 4 close to-the anti-rotation element 11 and extends through the slot 20 in the sleeve 9 und in the structure 3 towards the control board or is arranged between the drive unit 4 and the sleeve 9, in the latter case enabling the inclusion of all moveable parts 4, 5, 6, 13 including the flexprint 21 inside the sleeve 9, thereby forming an enclosed telescopic drive mechanism which can be provided as a module.

## Claims

1. A method of micro-dosing, in particular in a time-controlled manner, a liquid from a liquid reservoir (1) by axially moving a piston (2) arranged in said reservoir (1), thereby displacing liquid out of said reservoir (1), the method comprising the following steps:
a) providing a portable structure (3) for receiving the reservoir (1);
b) providing a drive unit (4) with a first end and a second end and with a drive motor (5), which drive unit (4) upon activation of said drive motor (5) is axially displaceable relative to said structure (3) ;
c) connecting said drive unit (4) at its second end to said piston (2) of said liquid reservoir (1) and securing said liquid reservoir (1) to said structure (3); and
d) activating said drive motor (5) to axially displace said drive unit (4) and a first member (6) relative to a second member (9) and to said structure (3) towards said piston (2) of said reservoir (1), thereby axially moving said piston (2) in said reservoir (1) and displacing liquid out of said reservoir (1),
wherein said axial displacement is effected by rotating, with said drive unit (4), said first member (6) around an axis of rotation (X), said first member (6) defining said axis of rotation (X) and being coupled to said drive unit (4) at the first end of said drive unit (4) and having an outer thread (7), the outer thread (7) being coaxially around said axis of rotation (X) and engaging a thread (8) of said second member (9) which is formed by or secured to said structure (3)
and
wherein between said drive unit (4) and said second member (9) an anti-rotation element (11) is arranged which prevents a rotation of said drive unit (4) relative to said second member (9) but permits axial movement of said drive unit (4) relative to said second member (9).

2. Method according to claim 1, wherein said thread (8) of said second member (9) is an inner thread (8) and said drive unit (4) is axially displaced from a retracted position, in which said second end of said drive unit (4) is closest to said inner thread (8) of said second member (9), to an extended position, in which said second end of said drive unit (4) is farthest away from said inner thread (8) of said second member (9), in such a manner that said first end of said drive unit (4) at least in the retracted position is at least partly circumferentially surrounded by said inner thread (8) of said second member (9).

3. Method according to one of the preceding claims, wherein the activity of said drive unit (4) is monitored by means of an encoder (10) arranged at said drive unit (4).

4. Method according to one of the preceding claims, wherein the forces exerted to said piston (2) of said liquid reservoir (1) are monitored with a force sensor (15).

5. Method according to one of the preceding claims, wherein the power for operating said drive motor (5) is supplied to said drive motor (5) via said anti- rotation element (11).

6. Method according to one of the preceding claims, wherein said drive motor (5) is activated by an electronic control system according to a specific activity profile, for the time-controlled dispensing of liquid from the liquid reservoir.

7. A portable device, in particular for performing the method according to one of the preceding claims, for micro-dosing of a liquid from a liquid reservoir (1) to be received inside the device by axially moving a piston (2) arranged in said reservoir (1) and thereby displacing liquid out of said reservoir (1), the device comprising:
a) a structure (3) for receiving the reservoir (1) and
b) a drive unit (4) with a first end and a second end and with a drive motor (5), which drive unit (4) is axially displaceable relative to said structure (3) by activating said drive motor (5),
wherein said drive unit (4) at its second end comprises coupling means (14) for coupling it in a releasable manner to said piston (2) of said liquid reservoir (1), in order to axially move said piston (2) in said reservoir (1) upon an axial movement of said drive unit (4), and further comprising:
c) a rotatable first member (6) defining an axis of rotation (X) and having an external thread (7) coaxially arranged around said axis of rotation (X);
and
d) a second member (9) formed by or secured to said structure (3) of said device, having a thread (8) which engages said outer thread (7) of said first member (6);
wherein said first member (6) is coupled to said drive unit (4) at the first end of said drive unit (4) in such a manner that, upon activation of said drive unit (4), said first member (6) is rotated by said drive unit (4) around its axis of rotation (X), thereby axially displacing said first member (6) and said drive unit (4) relative to said second member (9) and to said structure (3) and wherein between said drive unit (4) and said second member (9) there is arranged an anti-rotation element (11) which prevents a rotation of said drive unit (4) relative to said second member (49) but permits axial movement of said drive unit (4) relative to said second member (9).

8. The device according to claim 7, wherein said thread (8) of said second member (9) is an inner thread (8) and wherein said first member (6) and said drive unit (4), upon activation of said drive unit (4) and subsequent rotation of said first member (6) around its axis of rotation (X), can axially be displaced relative to said second member (9) from a retracted position, in which said second end of said drive unit (4) is closest to said inner thread (8) of said second member (9), to an extended position, in which said second end of said drive unit (4) is farthest away from said inner thread (8) of said second member (9), and wherein in said retracted position said second member (9) with its inner thread (8) at least partly circumferentially surrounds at least said first end of said drive unit (4).

9. The device according to claim 8, wherein in said retracted position more than half of the length of said drive unit (4) is at least partly circumferentially surrounded by said inner thread (8) of said second member (9).

10. The device according to one of the claims 8 to 9, wherein said second member (9) is a sleeve- like element with said internal thread (8) at its inside, out of which at one end thereof said second end of said drive unit (4) protrudes.

11. The device according to claim 10, wherein at said end of said second member (9), out of which said second end of said drive unit (4) protrudes, between said second member (9) and said drive unit (4) there are arranged sealing means (12) in order to prevent dirt or liquids to enter said second member (9).

12. The device according to one of the claims 7 to 11, wherein said drive unit (4) further comprises a gear (13) arranged between said drive motor (5) and said first member (6), and in particular, wherein said gear (13) forms said first end of said drive unit (4).

13. The device according to one of the claims 7 to 12, wherein said drive motor (5) has a drive shaft defining an axis of rotation (Y) and wherein this axis of rotation (Y) of said drive shaft and said axis of rotation (X) of said first member (6) are coaxially aligned.

14. The device according to one of the claims 7 to 13, wherein said drive unit (4) comprises an encoder (10) for monitoring the activity of said drive unit (4).

15. The device according to claim 14, wherein said encoder (10) is arranged at said second end of said drive unit (4), and in particular, wherein said encoder (10) is at least partially arranged inside a protrusion (14) which in operation is at least in part received in said piston (2) of said liquid reservoir (1).

16. The device according to one of claims 7 to 15, wherein the device is designed in such a manner that the power for operating said drive motor (5) is supplied via said anti-rotation elements (11) to said drive motor (5).

17. The device according to one of the claims 7 to 16, wherein the device comprises a force sensor (15) in order to permit in operation determination of the forces exerted to said piston (2) of said liquid reservoir (1).

18. The device according to one of the claims 7 to 17, wherein said axis of rotation (X) of said first member (6) is coaxially aligned with a central axis (Z) of said piston (2) of said liquid reservoir (1) which in operation is received in the device.

19. The device according to one of the claims 7 to 18, further comprising computerized control means for controlling said drive unit (4) in such a manner that a time-controlled dispensing of liquid is possible.

20. The device according to one of the claims 7 to 19, further comprising a liquid reservoir with a displacement piston.

## Patentansprüche

1. Verfahren zum Mikrodosieren, insbesondere auf eine zeitgesteuerte Weise, einer Flüssigkeit aus einem Flüssigkeitsreservoir (1) durch axiales Bewegen eines in dem Reservoir (1) angeordneten Kolbens (2), wodurch Flüssigkeit aus dem Reservoir (1) hinaus befördert wird, wobei das Verfahren folgende Schritte umfasst:
a) Bereitstellen einer tragbaren Struktur (3) zum Aufnehmen des Reservoirs (1);
b) Bereitstellen einer Antriebseinheit (4) mit einem ersten Ende und einem zweiten Ende und mit einem Antriebsmotor (5), wobei die Antriebseinheit (4) bei Betätigung des Antriebsmotors (5) im Verhältnis zu der Struktur (3) axial verschiebbar ist;
c) Verbinden der Antriebseinheit (4) an deren zweitem Ende mit dem Kolben (2) des Flüssigkeitsreservoirs (1) und Befestigen des Flüssigkeitsreservoirs (1) an der Struktur (3); sowie
d) Betätigen des Antriebsmotors (5) zum axialen Versetzen der Antriebseinheit (4) und eines ersten Bauteils (6) im Verhältnis zu einem zweiten Bauteil (9) und zu der Struktur (3) in Richtung des Kolbens (2) des Reservoirs (1), wodurch der Kolben (2) in dem Reservoir (1) axial bewegt wird und Flüssigkeit aus dem Reservoir (1) hinaus befördert wird,
wobei das axiale Verschieben durch Drehen des ersten Bauteils (6) mittels der Antriebseinheit (4) um eine Drehachse (X) bewirkt wird, wobei das erste Bauteil (6) die Drehachse (X) definiert und an dem ersten Ende der Antriebseinheit (4) an die Antriebseinheit (4) gekoppelt ist und ein Außengewinde (7) umfasst, wobei das Außengewinde koaxial um die Drehachse (X) herum angeordnet ist und mit einem Gewinde (8) des zweiten Bauteils (9), welches durch die Struktur (3) ausgebildet oder an diese fixiert ist, in Eingriff steht,
und
wobei zwischen die Antriebseinheit (4) und das zweite Bauteil (9) ein Drehblockierungselement (11) angeordnet ist, welches ein Drehen der Antriebseinheit (4) bezüglich des zweiten Bauteils (9) verhindert, jedoch axiale Bewegung der Antriebseinheit (4) bezüglich des zweiten Bauteils (9) gestattet.

2. Verfahren nach Anspruch 1, wobei das Gewinde (8) des zweiten Bauteils (9) ein Innengewinde (8) ist und die Antriebseinheit (4) aus einer eingefahrenen Position, in welcher das zweite Ende der Antriebseinheit (4) am dichtesten beim Innengewinde (8) des zweiten Bauteils (9) ist, derart axial in eine ausgefahrene Position verschoben wird, in welcher das zweite Ende der Antriebseinheit (4) am weitesten vom Innengewinde (8) entfernt ist, dass das erste Ende der Antriebseinheit (4) zumindest in der eingefahrenen Position von dem Innengewinde (8) des zweiten Bauteils (9) zumindest teilweise umlaufend umgeben ist.

3. Verfahren nach einem der vorhergehenden Ansprüche, wobei die Betätigung der Antriebseinheit (4) mittels eines an der Antriebseinheit (4) angeordneten Encoders (10) überwacht wird.

4. Verfahren nach einem der vorhergehenden Ansprüche, wobei die auf den Kolben (2) des Flüssigkeitsreservoirs (1) ausgeübten Kräfte mittels eines Kraftsensors (15) überwacht werden.

5. Verfahren nach einem der vorhergehenden Ansprüche, wobei die Energie zum Betreiben des Antriebsmotors (5) dem Antriebsmotor (5) über das Drehblockierungselement (11) zugeführt wird.

6. Verfahren nach einem der vorhergehenden Ansprüche, wobei der Antriebsmotor (5) durch ein elektronisches Steuersystem gemäß einem spezifischen Aktivierungsprofil zur zeitgesteuerten Abgabe einer Flüssigkeit aus dem Flüssigkeitsreservoir betätigt wird.

7. Tragbare Vorrichtung, insbesondere zum Ausführen des Verfahrens nach einem der vorhergehenden Ansprüche, zum Mikrodosieren einer Flüssigkeit aus einem in der Vorrichtung aufzunehmenden Flüssigkeitsreservoirs (1) durch axiales Bewegen eines in dem Reservoir (1) angeordneten Kolbens (2), und dadurch Befördern von Flüssigkeit aus dem Reservoir (1) hinaus, wobei die Vorrichtung Folgendes umfasst:
a) eine Struktur (3) zum Aufnehmen des Reservoirs (1) und
b) eine Antriebseinheit (4) mit einem ersten Ende und einem zweiten Ende und mit einem Antriebsmotor (5), wobei die Antriebseinheit (4) durch Betätigung des Antriebsmotors (5) im Verhältnis zu der Struktur (3) axial verschiebbar ist,
wobei die Antriebseinheit (4) an deren zweitem Ende Kupplungselemente (14) umfasst, um sie lösbar an den Kolben (2) des Flüssigkeitsreservoirs (1) zu koppeln, um den Kolben (2) in dem Reservoir (1) bei einer axialen Bewegung der Antriebseinheit (4) axial zu bewegen, und ferner Folgendes umfassend:
c) ein drehbares erstes Bauteil (6), welches eine Drehachse (X) definiert und ein koaxial um die Drehachse (X) herum angeordnetes Außengewinde (7) umfasst; sowie
d) ein zweites Bauteil (9), welches durch die Struktur (3) der Vorrichtung ausgebildet oder durch diese fixiert ist, ein Gewinde (8) umfassend, welches mit dem Außengewinde (7) des ersten Bauteils (6) in Eingriff steht;
wobei das erste Bauteil (6) an dem ersten Ende der Antriebseinheit (4) derart an die Antriebseinheit (4) gekoppelt ist, dass bei Betätigung der Antriebseinheit (4) das erste Bauteil (6) durch die Antriebseinheit (4) um seine Drehachse (X) gedreht wird, wodurch das erste Bauteil (6) und die Antriebseinheit (4) im Verhältnis zu dem zweiten Bauteil (9) und der Struktur (3) axial verschoben werden, und wobei zwischen der Antriebseinheit (4) und dem zweiten Bauteil (9) ein Drehblockierungselement (11) angeordnet ist, welches ein Drehen der Antriebseinheit (4) bezüglich des zweiten Bauteils (9) verhindert, jedoch eine axiale Bewegung der Antriebseinheit (4) bezüglich des zweiten Bauteils (9) gestattet.

8. Vorrichtung nach Anspruch 7, wobei das Gewinde (8) des zweiten Bauteils (9) ein Innengewinde (8) ist und wobei das erste Bauteil (6) und die Antriebseinheit (4), bei Betätigung der Antriebseinheit (4) und nachfolgendem Drehen des ersten Bauteils (6) um seine Drehachse (X) bezüglich des zweiten Bauteils (9) aus einer eingefahrenen Position, in welcher das zweite Ende der Antriebseinheit (4) am nächsten an dem Innengewinde (8) des zweiten Bauteils (9) ist, axial in eine ausgefahrene Position verschoben werden können, in welcher das zweite Ende der Antriebseinheit (4) am weitesten von dem Innengewinde (8) des zweitens Bauteils (9) entfernt ist, und wobei in der eingefahrenen Position das zweite Bauteil (9) mit seinem Innengewinde (8) zumindest das erste Ende der Antriebseinheit (4) zumindest teilweise umlaufend umgibt.

9. Vorrichtung nach Anspruch 8, wobei in der eingefahrenen Position mehr als die Hälfte der Länge der Antriebseinheit (4) durch das Innengewinde (8) des zweiten Bauteils (9) zumindest teilweise umlaufend umgeben ist.

10. Vorrichtung nach einem der Ansprüche 8 bis 9, wobei das zweite Bauteil (9) ein hülsenartiges Element mit dem Innengewinde (8) in seinem Innerem ist, aus welchem an einem seiner Enden das zweite Ende der Antriebseinheit (4) herausragt.

11. Vorrichtung nach Anspruch 10, wobei an dem Ende des zweiten Bauteils (9), aus welchem das zweite Ende der Antriebseinheit (4) herausragt, zwischen dem zweiten Bauteil (9) und der Antriebseinheit (4) Dichtungsmittel (12) angeordnet sind, um zu verhindern, dass Schmutz oder Flüssigkeiten in das zweite Bauteil (9) eindringen.

12. Vorrichtung nach einem der Ansprüche 7 bis 11, wobei die Antriebseinheit (4) ferner ein zwischen dem Antriebsmotor (5) und dem ersten Bauteil (6) angeordnetes Getriebe (13) umfasst, und wobei insbesondere das Getriebe (13) das erste Ende der Antriebseinheit (4) bildet.

13. Vorrichtung nach einem der Ansprüche 7 bis 12, wobei der Antriebsmotor (5) einen eine Rotationsachse (Y) definierende Abtriebswelle umfasst, und wobei die Drehachse (Y) des Antriebsschaftes und die Drehachse (X) des ersten Bauteils (6) zueinander koaxial sind:

14. Vorrichtung nach einem der Ansprüche 7 bis 13, wobei die Antriebseinheit (4) einen Encoder (10) zum Überwachen der Tätigkeit der Antriebseinheit (4) umfasst.

15. Verfahren nach Anspruch 14, wobei der Encoder (10) an dem zweiten Ende der Antriebseinheit (4) angeordnet ist und wobei insbesondere der Geber (10) zumindest teilweise in einem Vorsprung (14) angeordnet ist, welcher bei Betrieb zumindest teilweise in dem Kolben (2) des Flüssigkeitsreservoirs (1) aufgenommen ist.

16. Vorrichtung nach einem der Ansprüche 7 bis 15, wobei die Vorrichtung derart konzipiert ist, dass die Energie zum Betreiben des Antriebsmotors (5) dem Antriebsmotor (5) über die Drehblockierungselemente (11) zugeführt wird.

17. Vorrichtung nach einem der Ansprüche 7 bis 16, wobei die Vorrichtung einen Kraftsensor (15) umfasst, um bei Betrieb eine Bestimmung der auf den Kolben (2) des Flüssigkeitsreservoirs (1) ausgeübten Kräfte zu gestatten.

18. Vorrichtung nach einem der Ansprüche 7 bis 17, wobei die Drehachse (X) des ersten Bauteils (6) mit einer Mittelachse (Z) des Kolbens (2) des Flüssigkeitsreservoirs (1), welches bei Betrieb in der Vorrichtung aufgenommen wird, koaxial ausgerichtet ist.

19. Vorrichtung nach einem der Ansprüche 7 bis 18, ferner computergestützte Steuermittel zum derartigen Steuern der Antriebseinheit (4) umfassend, dass eine zeitgesteuerte Abgabe von Flüssigkeit möglich ist.

20. Vorrichtung nach einem der Ansprüche 7 bis 19, ferner ein Flüssigkeitsreservoir mit einem Verschiebekolben umfassend.

## Revendications

1. Procédé de microdosage, en particulier de manière commandée dans le temps, d'un liquide provenant d'un réservoir de liquide (1) par déplacement axial d'un piston (2) disposé dans ledit réservoir (1), ce qui déplace le liquide hors dudit réservoir (1), le procédé comprenant les étapes suivantes consistant à :
a) produire une structure portable (3) destinée à recevoir le réservoir (1) ;
b) produire une unité d'entraînement (4) comportant une première extrémité et une seconde extrémité et comportant un moteur d'entraînement (5), laquelle l'unité d'entraînement (4) lors de l'activation dudit moteur d'entraînement (5) est déplaçable axialement par rapport à ladite structure (3) ;
c) relier ladite unité d'entraînement (4), au niveau de sa seconde extrémité, audit piston (2) dudit réservoir de liquide (1) et fixer ledit réservoir de liquide (1) à ladite structure (3) ; et
d) activer ledit moteur d'entraînement (5) pour déplacer axialement ladite unité d'entraînement (4) et un premier élément (6) par rapport à un second élément (9) et à ladite structure (3) vers ledit piston (2) dudit réservoir (1), ce qui déplace axialement ledit piston (2) dans ledit réservoir (1) et déplace le liquide hors dudit réservoir (1),
ledit déplacement axial étant effectué par rotation, avec ladite unité d'entraînement (4), dudit premier élément (6) sur un axe de rotation (X), ledit premier élément (6) définissant ledit axe de rotation (X) et étant accouplé à ladite unité d'entraînement (4) au niveau de la première extrémité de ladite unité d'entraînement (4) et comportant un filetage extérieur (7), le filetage extérieur (7) étant disposé coaxialement audit axe de rotation (X) et s'engageant avec un filetage (8) dudit second élément (9) qui est formé par ladite structure (3) ou fixé à celle-ci,
et
entre ladite unité d'entraînement (4) et ledit second élément (9) étant disposé un élément anti-rotation (11) qui empêche la rotation de ladite unité d'entraînement (4) par rapport audit second élément (9), mais qi permet un mouvement axial de ladite unité d'entraînement (4) par rapport audit second élément (9).

2. Procédé selon la revendication 1, dans lequel ledit filetage (8) dudit second élément (9) est un filetage intérieur (8) et ladite unité d'entraînement (4) est déplacé axialement d'une position rétractée, dans laquelle ladite seconde extrémité de ladite unité d'entraînement (4) est plus proche dudit filetage intérieure (8) dudit seconde élément (9), à une position déployée dans laquelle ladite seconde extrémité de ladite unité d'entraînement (4) est la plus éloignée dudit filetage intérieur (8) dudit second élément (9), de telle sorte que ladite première extrémité de ladite unité d'entraînement (4), au moins dans la position rétractée, est entourée au moins partiellement sur la périphérie par ledit filetage intérieur (8) dudit second élément (9).

3. Procédé selon l'une des revendications précédentes, dans lequel l'activité de ladite unité d'entraînement (4) est surveillée au moyen d'un codeur (10) disposé au niveau de ladite unité d'entraînement (4).

4. Procédé selon l'une des revendications précédentes, dans lequel les forces exercées sur ledit piston (2) dudit réservoir de liquide (1) sont surveillées par un capteur de force (15).

5. Procédé selon l'une quelconque des revendications précédentes, dans lequel la puissance pour faire fonctionner ledit moteur d'entraînement (5) est fourni audit moteur d'entraînement (5) par l'intermédiaire dudit élément anti-rotation (11).

6. Procédé selon l'une des revendications précédentes, dans lequel ledit moteur d'entraînement (5) est activé par un système de commande électronique en fonction d'un profil d'activité spécifique pour distribuer, de façon commandée dans le temps, du liquide provenant du réservoir de liquide.

7. Dispositif portable, destiné en particulier à mettre en oeuvre le procédé selon l'une des revendications précédentes, pour micro-doser un liquide provenant d'un réservoir de liquide (1), destiné à être reçu à l'intérieur du dispositif, par déplacement axial d'un piston (2) disposé dans ledit réservoir (1), ce qui déplace le liquide hors dudit réservoir (1), le dispositif comprenant :
a) une structure (3) destinée à recevoir le réservoir (1) et
b) une unité d'entraînement (4) comportant une première extrémité et une seconde extrémité et comportant un moteur d'entraînement (5), laquelle unité d'entraînement (4) est déplaçable axialement par rapport à ladite structure (3) par activation dudit moteur d'entraînement (5),
ladite unité d'entraînement (4) comportant à sa seconde extrémité des moyens d'accouplement (14) destinés à l'accouplement, de manière libérable, audit piston (2) dudit réservoir de liquide (1), afin de déplacer axialement ledit piston (2) dans ledit réservoir (1) lors d'un mouvement axial de ladite unité d'entraînement (4), et comprenant en outre :
c) un premier élément rotatif (6) définissant un axe de rotation (X) et comportant un filetage extérieur (7) disposé coaxialement audit axe de rotation (X) ;
et
d) un second élément (9) formé par ladite structure (3) dudit dispositif, ou fixé à celle-ci, comportant un filetage (8) qui s'engage avec ledit filetage extérieur (7) dudit premier élément (6) ;
ledit premier élément (6) étant accouplé à ladite unité d'entraînement (4) au niveau de la première extrémité de ladite unité d'entraînement (4) de telle manière que, lors de l'activation de ladite unité d'entraînement (4), ledit premier élément (6) est mis en rotation par ladite unité d'entraînement (4) sur son axe de rotation (X), ce qui déplace axialement ledit premier élément (6) et ladite unité d'entraînement (4) par rapport audit second élément (9) et à ladite structure (3) et entre ladite unité d'entraînement (4) et ledit second élément (9) étant disposé un élément anti-rotation (11) qui empêche la rotation de ladite unité d'entraînement (4) par rapport audit second élément (9), mais qui permet un mouvement axial de ladite unité d'entraînement (4) par rapport audit second élément (9).

8. Dispositif selon la revendication 7, dans lequel ledit filetage (8) dudit second élément (9) est un filetage intérieur (8) et dans lequel ledit premier élément (5) et ladite unité d'entraînement (4), lors de l'activation de ladite unité d'entraînement (4) et de la rotation subséquente dudit premier élément (6) sur son axe de rotation (X), peut être déplacée axialement par rapport audit second élément (9) d'une position rétractée, dans laquelle ladite seconde extrémité de ladite unité d'entraînement (4) est plus proche dudit filetage intérieur (8) dudit second élément (9), vers une position déployée dans laquelle ladite seconde extrémité de ladite unité d'entraînement (4) est la plus éloignée de dudit filetage intérieur (8) dudit second élément (9), et dans lequel, dans ladite position rétractée, ledit second élément (9) pourvu de son filetage intérieur (8) entoure au moins partiellement sur la périphérie au moins ladite première extrémité de ladite unité d'entraînement (4).

9. Dispositif selon la revendication 8, dans lequel dans ladite position rétractée plus de la moitié de la longueur de ladite unité d'entraînement (4) est entourée au moins partiellement sur la périphérie par ledit filetage intérieur (8) dudit second élément (9).

10. Dispositif selon l'une des revendications 8 à 9, dans lequel ledit second élément (9) est un élément en forme de manchon qui comporte à l'intérieur ledit filetage intérieur (8) à une des extrémités duquel ladite seconde extrémité de ladite unité d'entraînement (4) fait saillie.

11. Dispositif selon la revendication 10, dans lequel, au niveau de ladite extrémité dudit second élément (9) de laquelle ladite seconde extrémité de ladite unité d'entraînement (4) fait saillie, des moyens d'étanchéité (12) sont disposés entre ledit second élément (9) et ladite unité d'entraînement (4) afin d'empêcher des souillures ou des liquides de pénétrer dans ledit second élément (9).

12. Dispositif selon l'une des revendications 7 à 11, dans lequel ladite unité d'entraînement (4) comprend en outre un engrenage (13) disposé entre ledit moteur d'entraînement (5) et ledit premier élément (6), et en particulier, dans lequel ledit engrenage (13) forme ladite première extrémité de ladite unité d'entraînement (4).

13. Dispositif selon l'une des revendications 7 à 12, dans lequel ledit moteur d'entraînement (5) a un arbre d'entraînement (6) définissant un axe de rotation (Y), le axe de rotation (Y) de ledit arbre d'entraînement (6) et ledit axe de rotation (X) de ledit premier élément (6) sont alignée de manière coaxiale.

14. Dispositif selon l'une quelconque des revendications 7 à 13, dans lequel ladite unité d'entraînement (4) comprend un codeur (10) destiné à surveiller l'activité de ladite unité d'entraînement (4).

15. Dispositif selon l'une des revendications 14, dans lequel ledit codeur (10) est disposé au niveau de ladite seconde extrémité de ladite unité d'entraînement (4), et en particulier, dans lequel ledit codeur (1 0) est au moins partiellement disposé à l'intérieur d'une saillie (14) qui, en fonctionnement, est au moins en partie reçue dans ledit piston (2) dudit réservoir de liquide (1).

16. Dispositif selon l'une des revendications 7 à 15, dans lequel le dispositif est conçu de telle manière que la puissance pour faire fonctionner ledit moteur d'entraînement (5) est fournie audit moteur d'entraînement (5) par le bais desdits éléments anti-rotation (11).

17. Dispositif selon l'une quelconque des revendications 7 à 15, dans lequel le dispositif comprend un capteur de force (15) permettant de détermination en fonctionnement des forces exercées sur ledit piston (2) dudit réservoir de liquide (1).

18. Dispositif selon l'une des revendications 7 à 17, dans lequel ledit axe de rotation (X) dudit premier élément (6) est aligné coaxialement avec un axe central (Z) dudit piston (2) dudit réservoir de liquide (1) qui, en fonctionnement, est reçu dans le dispositif.

19. Dispositif selon l'une des revendications 7 à 18, comprenant en outre des moyens de commande informatisé destinés à commander ladite unité d'entraînement (4) de telle manière à permettre une distribution de liquide commandée dans le temps.

20. Dispositif selon l'une des revendications 7 à 19, comprenant en outre un réservoir de liquide comportant un piston de déplacement.
